# EUROPEAN PATENT APPLICATION

(11) **EP 4 272 732 A1**
(43) Date of publication of application: **08.11.2023**
(21) Application number: 21915805.2
(22) Date of filing: 29.12.2021
(51) Int. Cl.: A61K 9/14, A61K 31/454, A61K 47/60, A61K 9/20, A61K 9/08, A61P 31/10, C07D 401/06

(54) **EFINACONAZOLE ORAL COMPOSITION**

(30) Priority: 29.12.2020 KR 20200186819
(71) Applicant: Daebong LS Co., Ltd., Namdong-gu Incheon 21697 (KR)
(72) Inventor: PARK, Eun Ju, Hwaseong-si Gyeonggi-do 18425 (KR); JI, Hyun, Siheung-si Gyeonggi-do 14923 (KR); PARK, Jin Oh, Seoul 06276 (KR); PARK, Suck Yong, Seongnam-si Gyeonggi-do 13474 (KR); BANG, Min Hyuk, Suwon-si Gyeonggi-do 16353 (KR)
(74) Representative: Ter Meer Steinmeister & Partner
(86) International application number: PCT/KR2021/020112
(87) International publication number: WO 2022/146007

(57) **Abstract**

The present invention relates to a composition for oral use including a co-formed product of efinaconazole and polyethylene glycol and a pharmaceutically acceptable additive. The composition of the present invention has high stability and bioavailability and is thus very effective when administered.

## Description

### Technical Field

The present invention relates to a composition for oral use including efinaconazole that has high stability and bioavailability and is thus very effective when administered.

### Background Art

Efinaconazole is a triazole compound with proven therapeutic activity for onychomycosis. Efinaconazole exhibits antifungal activity by inhibiting the synthesis of ergosterol, an essential constituent of the fungal cell membrane.

Jublia^{™} is a currently commercially available product for efinaconazole. Jublia employs efinaconazole as a major ingredient and is used to treat onychomycosis caused by dermatophytes. Jublia received approval for use in South Korea by the Korean Ministry of Food and Drug Safety and was launched in South Korea in May 2017.

Jublia is a formulation for topical application. Topical antifungals are relatively free from the disadvantages of oral antifungals, such as skin rash, hepatotoxicity, and digestive system side effects, and exhibit high medication compliance and preference.

Formulations useful for topical delivery of efinaconazole and other triazole antifungal drugs for the treatment of onychomycosis are described, for example, in U.S. Patent No. 8,486,978 ("Patent Document 1").

However, formulations for topical application may be rather insufficient in inhibiting deep-rooted fungi due to the ability of fungi to penetrate the outer skin or nail and are somewhat limited in inhibiting and preventing fungi that spread widely throughout the entire body because they should be applied to lesions without leaving unapplied lesions.

Indeed, the reason why fungal diseases such as tinea manus, tinea pedis, and onychomycosis tend to recur is also closely related to the above-described limitations of formulations for topical application. That is, although formulations for topical application work well against fungi residing on the surface, they fail to work at desired effective concentrations to reach depths at which fungi are completely killed.

Thus, it is important to develop dosage forms (e.g., injections and tablets, solutions, capsules, and powders for oral use) that can deliver antifungal ingredients to target tissues through blood. It is particularly requested to develop oral formulations that are easy to take and have high medication compliance and preference.

However, efinaconazole has problems associated with uptake and bioavailability when administered orally due to its relatively low water solubility (~0.61 mg/mL).

Efinaconazole-containing solutions have the problem of poor stability, that is, they tend to discolor within short storage periods, resulting in composition colors ranging from yellow to deep red or brown. In an attempt to solve this stability problem, U.S. Patent No. 9,662,394 discloses a liquid or semi-solid composition including a specific chelating agent, an antioxidant, and acids, specifically ethylenediaminetetraacetic acid (EDTA) or a salt thereof, butylated hydroxytoluene (BHT), and citric acid.

Therefore, a highly bioavailable and stable oral formulation including efinaconazole needs to be developed.

For reference, the following prior art documents are cited herein and are hereby incorporated by reference in their entirety.

(Patent Document 1) U.S. Patent No. 8,486,978 B2 (July 16, 2013)

(Patent Document 2) U.S. Patent No. 9,662,394 B2 (May 30, 2017)

(Patent Document 3) U.S. Patent No. 5,620,994 A (April 15, 1997)

### Detailed Description of the Invention

### Problems to be Solved by the Invention

The present invention is intended to solve the problems of the prior art and to provide a highly bioavailable and stable oral formulation including efinaconazole.

### Means for Solving the Problems

The present invention has been made in an effort to solve the problems of the prior art.

The present invention provides a pharmaceutical composition for oral use including a co-formed product of efinaconazole and polyethylene glycol and a pharmaceutically acceptable additive.

The pharmaceutical composition of the present invention may be formulated into a tablet or solution.

The pharmaceutical composition of the present invention may include 0.6 to 2040 mg of the efinaconazole.

The weight ratio of the efinaconazole to the polyethylene glycol in the co-formed product may be 1:1.

The pharmaceutically acceptable additive may be selected from the group consisting of excipients, binders, disintegrants, surfactants, lubricants, colorants, and mixtures thereof.

The excipients may be lactose hydrate, microcrystalline cellulose, microcrystalline cellulose-lactose, methyl cellulose, ethyl cellulose, hydroxyethyl cellulose, hydroxypropyl cellulose, hydroxypropyl methyl cellulose, carboxymethyl cellulose salts, other substituted and unsubstituted celluloses, corn starch, pregelatinized starch, lactose, anhydrous lactose, sugar alcohols, sucrose, xylitol, acrylate polymers and copolymers, dextrates, dextrin, dextrose, maltodextrin, pectin, gelatin, inorganic diluents, and mixtures thereof.

The binders may be polyvinyl alcohol-polyethylene glycol graft copolymers, polyvinylpyrrolidone vinyl acetate, ethyl cellulose, hydroxypropyl cellulose, hydroxypropyl methyl cellulose, carboxymethyl cellulose, methyl cellulose, polyvinyl alcohol, polyvinylpyrrolidone, polyacrylic acid, gelatin, propylene glycol, sodium alginate, and mixtures thereof.

The disintegrants may be croscarmellose sodium, crospovidone, carboxymethyl cellulose salts, hydroxypropyl cellulose, microcrystalline cellulose, pregelatinized starch, sodium starch, glycolate, sodium starch glycolate, and mixtures thereof.

The lubricants may be magnesium stearate, sodium stearyl fumarate, glyceryl behenate, and mixtures thereof.

The pharmaceutical composition of the present invention may be used to prevent or treat a fungal infection.

The fungal infection may be onychomycosis.

### Effects of the Invention

The pharmaceutical composition of the present invention is suitable for oral administration due to its high stability and bioavailability.

### Brief Description of the Drawings

Fig. 1 is a powder XRD pattern of a co-crystal of efinaconazole and polyethylene glycol.
Fig. 2 is a DSC thermogram of a co-crystal of efinaconazole and polyethylene glycol.
Fig. 3 is a powder XRD pattern of a crystalline form of efinaconazole.
Fig. 4 is a DSC thermogram of a crystalline form of efinaconazole.
Fig. 5 shows graphs comparing the stability of a crystalline form of efinaconazole with that of a co-crystal of efinaconazole and polyethylene glycol.
Fig. 6 shows a time-blood voriconazole concentration curve in a preclinical experiment.
Fig. 7 shows time-blood efinaconazole concentration curves in a preclinical experiment.

### Best Mode for Carrying out the Invention

The present invention will now be described in detail.

The present invention is directed to a pharmaceutical composition for oral use including a co-formed product of efinaconazole and polyethylene glycol and a pharmaceutically acceptable additive.

A co-formed product is composed of two or more components and exhibits unique physical properties different from those of the individual components, just like a single material. Co-formed products are divided into co-amorphous products and co-crystals.

The term "co-crystal" refers to a crystal structure of two or more different molecules in a specific stoichiometric ratio within one crystal lattice. Intermolecular bonds in co-crystals are distinguished from those in salts and mixtures.

A co-crystal is a crystalline solid that is rich in functional groups (for example, O, OH, and N) capable of hydrogen bonding or has a new crystal structure through hydrogen bonding with a coformer in a regular ratio.

The term "coformer" refers to an inactive molecule constituting a co-crystal of a drug. In the present invention, polyethylene glycol is preferably selected as the conformer.

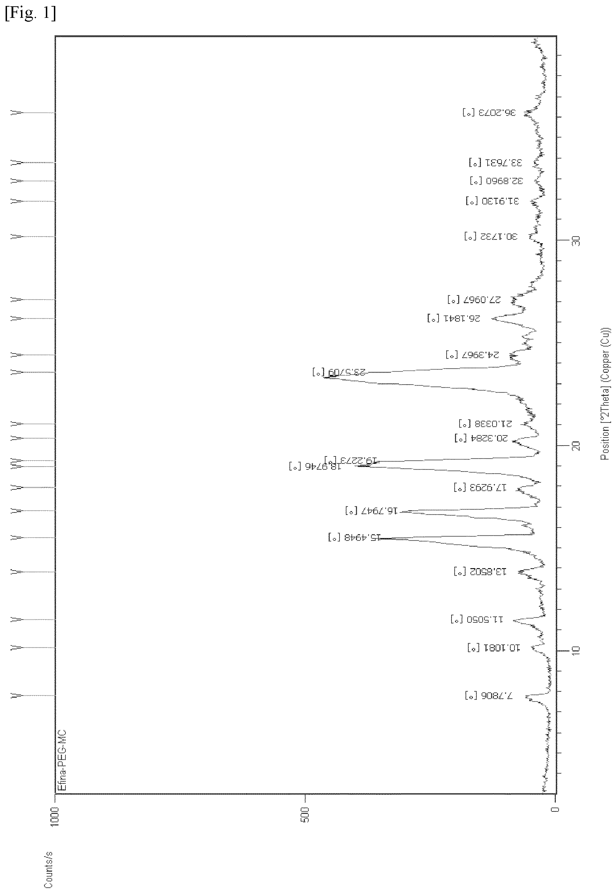

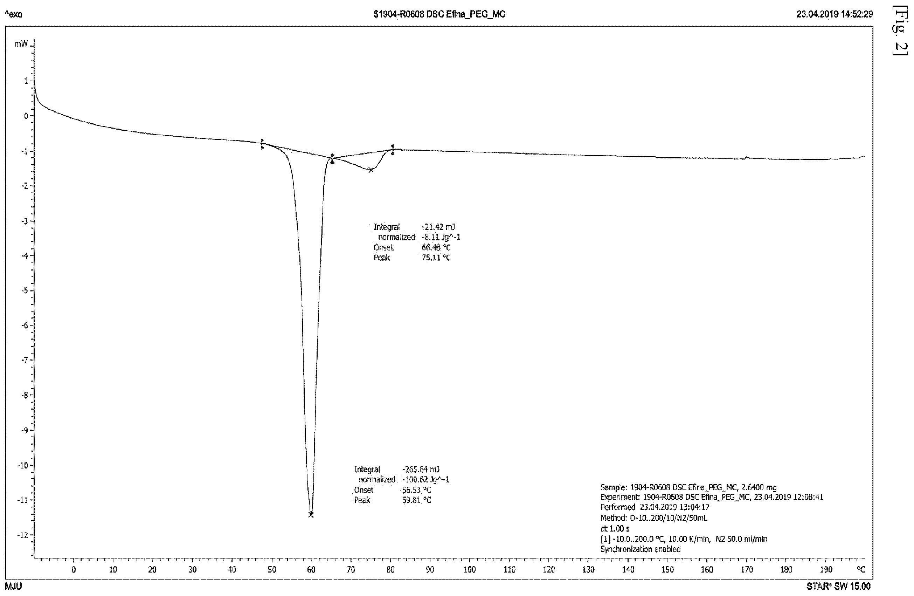

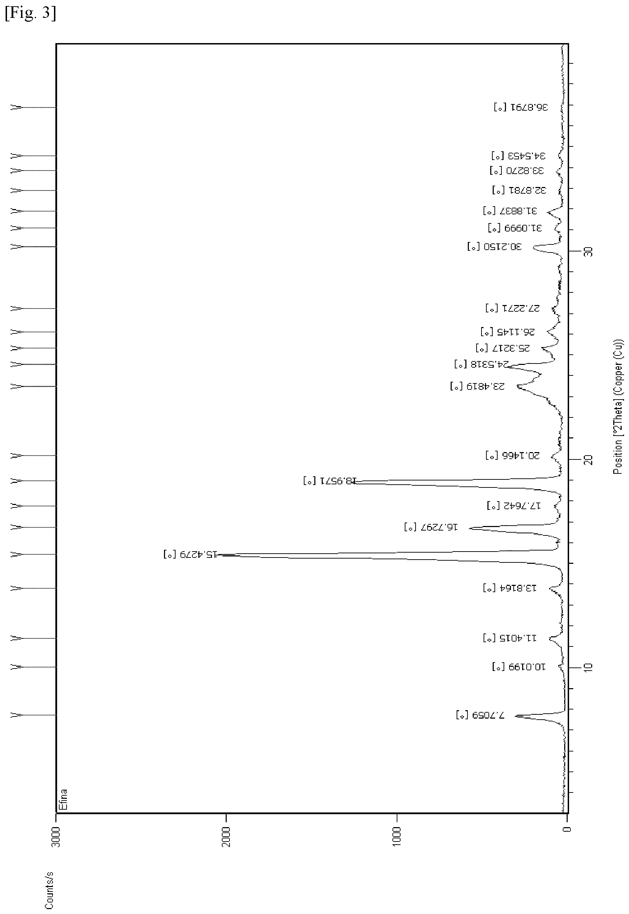

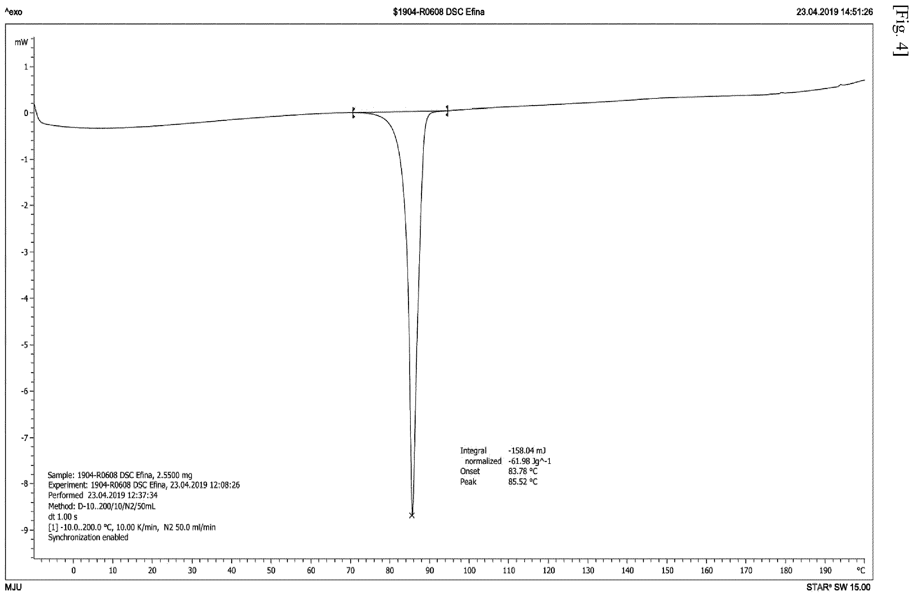

A powder XRD pattern and a DSC thermogram of a co-crystal of efinaconazole and polyethylene glycol are shown in Figs. 1 and 2, respectively. A XRD pattern and a DSC thermogram of a crystalline form of efinaconazole are shown in Figs. 3 and 4, respectively.

Comparative analysis of Figs. 1 to 4 shows that the co-formed product of efinaconazole exhibits a completely different tendency from that of the crystalline form of efinaconazole.

That is, the co-formed product of efinaconazole and polyethylene glycol has a crystal structure that exhibits characteristic peaks at diffraction angles (2θ I/I0 > 10%) of 7.78°, 11.50°, 13.85°, 15.49°, 16.79°, 18.97°, 19.22°, 23.57°, 26.18°, and 27.09° in its powder XRD pattern. The differential scanning calorimetry (DSC) thermogram of the co-formed product of efinaconazole and polyethylene glycol has maximum endothermic peaks at 61.62 °C and 78.78 °C. The XRD pattern and DSC thermogram of the co-formed product of efinaconazole and polyethylene glycol are distinctly different from those of the crystalline form of efinaconazole.

Therefore, it can be concluded that the co-formed product of efinaconazole is a novel substance having physicochemical properties entirely different from those of existing crystalline forms of efinaconazole.

Since efinaconazole is a somewhat unstable drug, its stability needs to be improved for use as an active ingredient of pharmaceutical compositions. The formation of the co-formed product can increase the stability of efinaconazole.

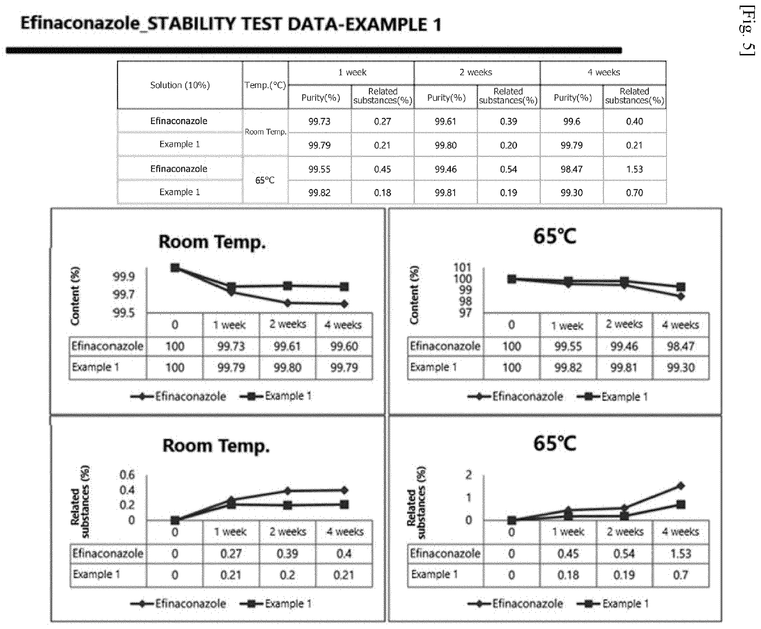

That is, the co-formed product of efinaconazole has excellent stability, as shown in Fig. 5.

Furthermore, the co-formed product of efinaconazole can increase the solubility and dissolution rate of the drug, with the result that the uptake of the drug into the body is changed, resulting in a change in bioavailability.

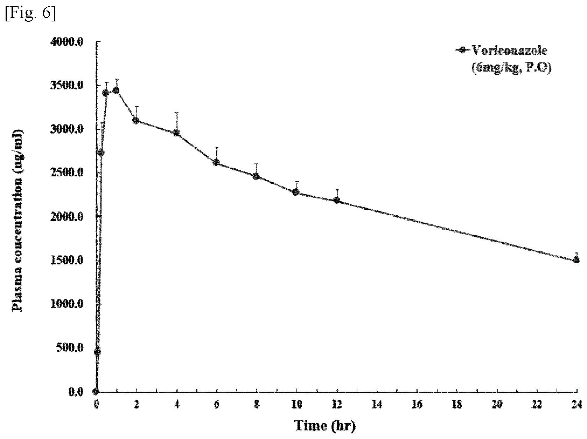

That is, the co-formed product of efinaconazole has excellent bioavailability, as shown in Fig. 6.

Based on these features, the co-formed product of efinaconazole meets all requirements (that is, requirements in terms of chemical purity of the crystal, stability to a solution and temperature, and bioavailability when administered orally) for use as an active ingredient of the pharmaceutical composition.

The stability or bioavailability of the co-formed product of efinaconazole will be more fully understood with reference to the Examples section that follows.

The pharmaceutical composition of the present invention includes a prophylactically or therapeutically effective amount of the efinaconazole as an active ingredient. The amount of the active ingredient is determined based on safety and effectiveness data. In some embodiments, the therapeutically effective amount may be from about 0.01 to about 30 mg/kg. In some specific embodiments, the therapeutically effective amount may be from about 0.01 to about 6 mg/kg. Thus, the content of efinaconazole in a formulation for oral administration may be 0.6 to 2040 mg or 0.6 to 410 mg but is not necessarily limited thereto. The efinaconazole content may vary depending on clinical findings.

The pharmaceutically acceptable additive may be selected from, but not limited to, pharmaceutical additives that are obvious and widely known to those skilled in the art. Examples of such pharmaceutically acceptable additives include stabilizers, surfactants, lubricants, solubilizers, buffers, sweeteners, bases, adsorbents, bitterants, binders, suspending agents, curing agents, antioxidants, brighteners, flavors, flavoring agents, pigments, coating agents, wetting agents, wettability control agents, fillers, defoamers, refrigerants, masticatories, antistatic agents, colorants, sugar-coated agents, isotonic agents, softeners, emulsifiers, pressure-sensitive adhesives, thickeners, blowing agents, pH adjusting agents, excipients, dispersants, disintegrants, waterproofing agents, antiseptics, preservatives, dissolution aids, solvents, and fluidizers. More specifically, the pharmaceutically acceptable additive may be selected from the group consisting of excipients, binders, disintegrants, surfactants, lubricants, colorants, and mixtures thereof.

Examples of the excipients include lactose hydrate, microcrystalline cellulose, microcrystalline cellulose-lactose, methyl cellulose, ethyl cellulose, hydroxyethyl cellulose, hydroxypropyl cellulose, hydroxypropyl methyl cellulose, carboxymethyl cellulose salts, other substituted and unsubstituted celluloses, corn starch, pregelatinized starch, lactose, anhydrous lactose, sugar alcohols, sucrose, xylitol, acrylate polymers and copolymers, dextrates, dextrin, dextrose, maltodextrin, pectin, gelatin, and inorganic diluents.

Examples of the binders include polyvinyl alcohol-polyethylene glycol graft copolymers, polyvinylpyrrolidone vinyl acetate, ethyl cellulose, hydroxypropyl cellulose, hydroxypropyl methyl cellulose, carboxymethyl cellulose, methyl cellulose, polyvinyl alcohol, polyvinylpyrrolidone, polyacrylic acid, gelatin, propylene glycol, and sodium alginate.

Examples of the disintegrants include croscarmellose sodium, crospovidone, carboxymethyl cellulose salts, hydroxypropyl cellulose, microcrystalline cellulose, pregelatinized starch, sodium starch, glycolate, and sodium starch glycolate.

Examples of the surfactants include sodium dodecyl sulfonate (SDS), sodium lauryl sulfate (SLS), Pluronic F127, Pluronic F68, poly(ethylene glycol) (PEG), polyoxyethylene sorbitan monooleate (Tween 80), Poloxamer (Lutrol F68), polyethylene glycol-15-hydroxystearate (Solutol HS15), hydroxypropyl methyl cellulose (HPMC), poly(N-vinyl-2-pyrrolidone) (PVP), Silwet L77, poly(vinylalcohol) (PVA), bovine serum albumin (BSA), and sucrose fatty acid esters.

Examples of the lubricants include, but are not necessarily limited to, magnesium stearate, sodium stearyl fumarate, and glyceryl behenate. The lubricants may be, but not limited to, pharmaceutical additives that are obvious and widely known to those skilled in the art.

The composition of the present invention can be used to prevent or treat a fungal infection. Specific examples of indications for the fungal infection include, but are not limited to, candidal vaginitis, tinea versicolor, tinea corporis caused by dermatophytes, tinea capitis (jock itch), tinea manus, tinea pedis, oral candidiasis, fungal keratitis, onychomycosis, aspergillosis, candidiasis, candiduria, candidemia, cryptococcosis (including cryptococcal meningitis), paracoccidioidomycosis, systemic fungal infections, seborrheic dermatitis, and dandruff caused by yeast.

The present invention will be more specifically explained with reference to the following examples. However, it is to be noted that these examples are not intended to limit the scope of the invention.

### EXAMPLES

### Example 1 - Preparation of co-crystal of efinaconazole and polyethylene glycol (1:1 ratio)

Efinaconazole (1 g) was dissolved in acetonitrile (5 mL) with stirring at room temperature. Polyethylene glycol-6000 (1 g) was added to the solution, followed by stirring for 1 h. The solvent was distilled off under reduced pressure. Spontaneous formation of a co-crystal of efinaconazole and polyethylene glycol-6000 was observed within a short time. After drying under vacuum, the crystal was recovered in a yield of 95%. The purity of the crystal was 99.5%. A powder X-ray diffraction (XRD) spectrum and a differential scanning calorimetry thermogram of the co-crystal of efinaconazole and polyethylene glycol-6000 are shown in Figs. 1 and 2, respectively.

### Example 2 - Preparation of oral liquid formulation

The co-crystal prepared in Example 1 and a solution of methylcellulose (0.5%) dispersed in distilled water containing surfactant (1%) were vortexed and sonicated to prepare a homogeneous oral liquid formulation. The components and their amounts are specifically shown in Table 1.

**[Table 1]**

| Application category | | Component | Amount allowed |
|---|---|---|---|
| 1 | Major ingredient | Example 1 | 69 mg |
| 2 | Excipient | Methyl cellulose | 50 mg |
| 3 | Surfactant | Tween 80 | 100 µl |
| 4 | Solvent | Purified water | 10 ml |

### Example 3 - Preparation of oral tablets

The co-crystal prepared in Example 1 and excipients were kneaded, and disintegrants and lubricants were sequentially added thereto. The resulting particles were milled and compressed into uncoated tablets. The components and their amounts are specifically shown in Table 2.

**[Table 2]**

| Application Category | | Component | Amount allowed | Amount used | | |
|---|---|---|---|---|---|---|
| | | | 1T (mg) | 600 | T/g | Proportion (%) |
| 1 | Major ingredient | Example 1 | 7.50 | 4.50 | | 7.50% |
| 2 | Excipient | Lactose hydrate | 50.00 | 30.00 | | 50.00% |
| 3 | Excipient | Microcrystalline cellulose | 31.00 | 18.60 | | 31.00% |
| 5 | Disintegrant | Low-substituted hydroxypropyl cellulose | 5.00 | 3.00 | | 5.00% |
| 6 | Disintegrant | Crospovidone | 5.00 | 3.00 | | 5.00% |
| 7 | Lubricant | Colloidal silica | 0.50 | 0.30 | | 0.50% |
| 8 | Lubricant | Magnesium stearate | 1.00 | 0.60 | | 1.00% |

### Example 4 - Preparation of oral capsules

The milled particles before tableting in Example 3 were filled in gelatin capsules to prepare capsules.

### Test Examples

### [Stability test]

The stability of the co-crystal of efinaconazole to temperature and a solution was evaluated and compared with that of efinaconazole by the following experimental method.

### * Experimental method

A co-crystalline form of efinaconazole was prepared. After each of the co-crystalline form and the co-crystal prepared in Example 1 was dissolved in a solution and allowed to stand at different temperatures (at room temperature and in an oven at 65 °C) for different periods of time (1 week, 2 weeks, and 4 weeks), its purities were determined by high-performance liquid chromatography (HPLC).

The results are shown in Table 3 and Fig. 5.

**[Table 3]**

| Solution (10%) | Temp. (°C) | **After 1 week** | | **After 2 weeks** | | **After 4 weeks** | |
|---|---|---|---|---|---|---|---|
| | | HPLC (%) | Related substances (%) | HPLC (%) | Related substances (%) | HPLC (%) | Related substances (%) |
| Efinaconazole | Room temp. | 99.73 | 0.27 | 99.61 | 0.39 | 99.60 | 0.40 |
| Example 1 | | 99.79 | 0.21 | 99.80 | 0.20 | 99.79 | 0.21 |
| Efinaconazole | 65 | 99.55 | 0.45 | 99.46 | 0.54 | 98.47 | 1.53 |
| Example 1 | | 99.80 | 0.18 | 99.81 | 0.19 | 99.30 | 0.70 |

As can be seen from the results in Table 3 and Fig. 5, the co-crystal of efinaconazole was more stable at the temperatures and the solution than the crystalline form of efinaconazole, thus being more useful in the preparation of medicaments. In addition, the co-crystal of efinaconazole is considered as a novel substance that meets all requirements for use as an active ingredient of a pharmaceutical composition.

### [Pre-clinical trials]

24 male beagle dogs were divided into 4 groups, 6 animals per group. Voriconazole as a control drug was once administered to the animals via an oral route under fasting conditions. Efinaconazole as a test drug was once administered to the animals via intravenous and oral routes under fasting conditions. Thereafter, the pharmacokinetic (PK) profiles of the drugs were determined to calculate PK parameters of the drugs and evaluate the pharmacokinetics of the drugs. Specific test conditions and methods are as follows.

### 1. Preparation of test materials

For oral administration, the doses of voriconazole (oral administration group G1), efinaconazole (oral administration group G2), and the co-crystal of efinaconazole prepared in Example 1 (oral administration group G3 - efinaconazole copolymer) were calculated based on the body weights of the animals. The test substances were weighed with an electronic balance. Each of the weighed test substances was placed in a glass container and 0.5% methylcellulose in a 1% Tween 80 solution (distilled water) as an excipient was added thereto. The mixture was vortexed and sonicated to prepare a homogeneous test material.

For intravenous administration, the doses of efinaconazole (intravenous administration group G1) were calculated based on the body weights of the animals. To the test substance was added PEG400 as an excipient whose volume corresponded to 30% of the total volume of a final test material. The mixture was vortexed and sonicated for complete dissolution. To the solution was added ethanol in an amount corresponding to 20% of the total volume of the test material, followed by homogenization by sonication. Then, 0.9% saline was added in an amount corresponding to 50% of the total volume of the test material, followed by vortexing and sonication to *in situ* prepare the test material in a homogeneous state.

### 2. Administration

- Routes of administration: Intravenous (IV) and oral (PO)
- Doses: G1 (Voriconazole) - 6 mg/5 ml/kg (PO); G2 (Efinaconazole) - 6 mg/5 ml/kg (PO); G3 (Efinaconazole - Polymer) - 6 mg/5 ml/kg (PO); G1 (Efinaconazole) - 0.5 mg/1 ml/kg (IV)
- Frequency of administration: Single dose (once daily) - (QD)

The compositions of the test materials administered are shown in Table 4.

**[Table 4]**

| • Oral administration | | | | | | | |
|---|---|---|---|---|---|---|---|
| Group | Substance | Dose (mg/kg) | Dosage volume (ml/kg) | Route of administration | Frequency of administration | Number of animals (n) | Animal Nos. |
| G1 | Voriconazole | 6 | 5 | P.O | 1 | 6 | 1-6 |
| G2 | Efinaconazole | 6 | 5 | P.O | 1 | 6 | 7-12 |
| G3 | Efinaconazole co-crystal (copolymer) | 6 | 5 | P.O | 1 | 6 | 13-18 |

| • Intravenous administration | | | | | | | |
|---|---|---|---|---|---|---|---|
| Group | Substance | Dose (mg/kg) | Dosage volume (ml/kg) | Route of administration | Frequency of administration | Number of animals (n) | Animal Nos. |
| G1 | Efinaconazole | 0.5 | 1 | I.V | 1 | 6 | 1-6 |

### 3. Test results

Animal Nos. 1-6, Nos. 7-12, and Nos. 13-18 were assigned to oral administration groups G1, G2, and G3, respectively, and animal Nos. 1-6 were assigned to intravenous administration group G1. On the test day, the average body weights of oral administration groups G1, G2, and G3 were measured to be 11.5±1.2 kg, 11.5±1.3 kg, and 11.5±1.0 kg, respectively, and that of intravenous administration group G1 was measured to be 11.6±1.6 kg.

During testing, no abnormal symptoms (including vomiting, limpness, diarrhea, and behavioral abnormalities) associated with the administration of the test materials were observed. There were no cases of death and blood collection proceeded smoothly over the entire test period. 4 ml of blood was collected from each point and centrifuged. 500 µl of the plasma supernatant was dispensed in a 1.5 ml EP tube and the remaining plasma was dispensed into a set of 1.5 ml EP tubes. The samples were stored in a deep freezer at -80 °C. After completion of the testing, the blood collection records, sample information certificates, dispensing records, clinical symptom observation records, medication/blood collection results, and test samples (plasma samples) were sent to a specialized analysis agency.

After storage in a freezer, the samples were analyzed by LC/MS/MS and their pharmacokinetic parameters were calculated in a noncompartmental manner using "Phoenix WinNonlin (Version 8.2 or higher, Certara L.P)" software.

For analysis of the plasma samples, animal Nos. 1-6 were assigned to voriconazole group G1 (6 mg/kg, P.O) and animal Nos. 1-6, Nos. 7-12, and Nos. 13-18 were assigned to efinaconazole groups G2 (0.6 mg/kg, I.V), G3 (6 mg/kg, P.O), and G4 (copolymer, 6 mg/kg, P.O), respectively, as shown in Table 5.

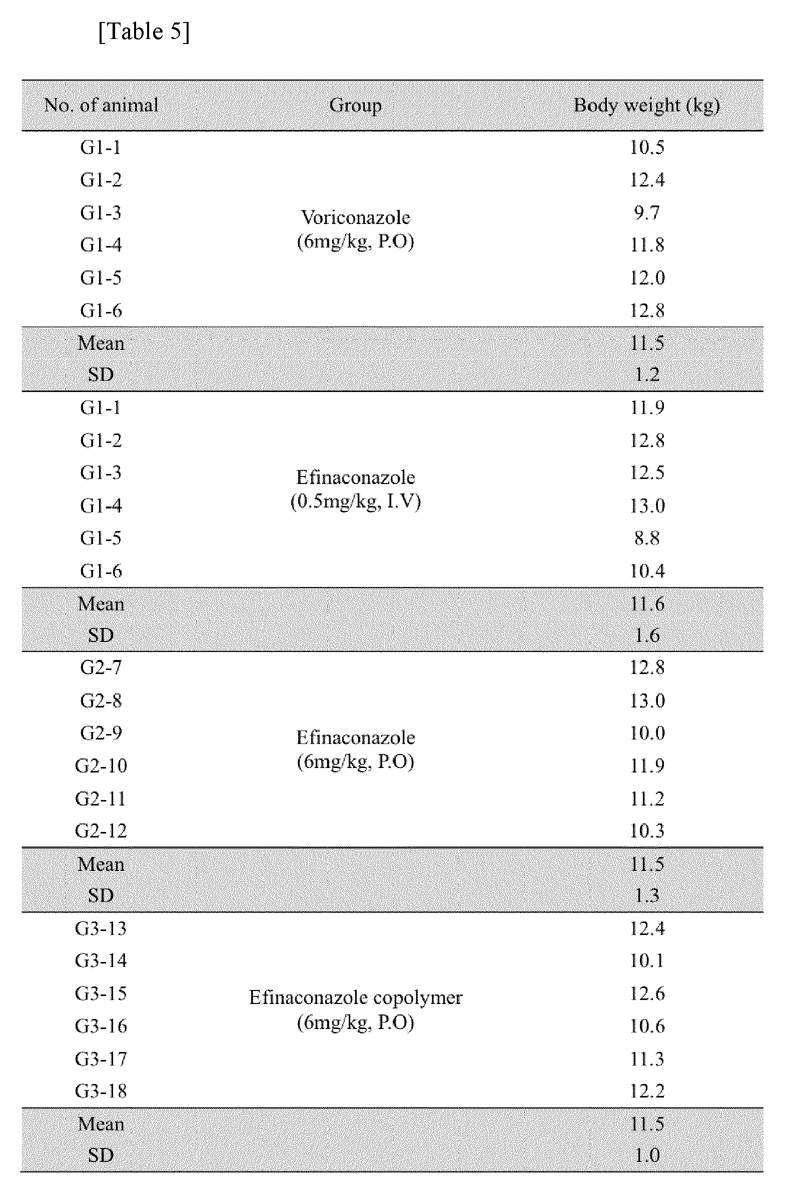

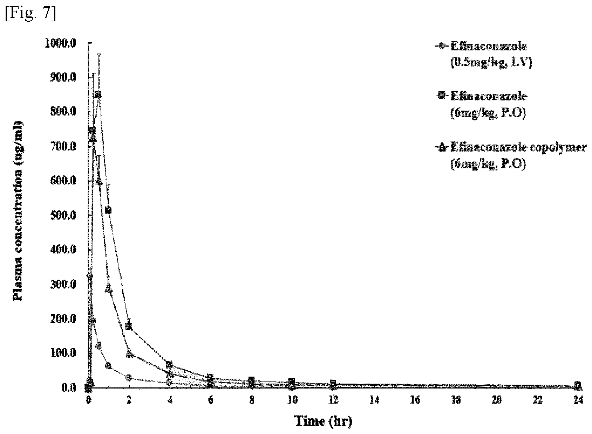

After the drugs were once administered to the 4 groups, 6 animals per group, a total of 24 test samples were analyzed. The trends of blood concentrations of voriconazole and efinaconazole are shown in Figs. 6 and 7 and Tables 6 and 7. Peaks detected in some of the test samples were below the limit of quantification and were expressed as BLQ.

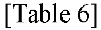

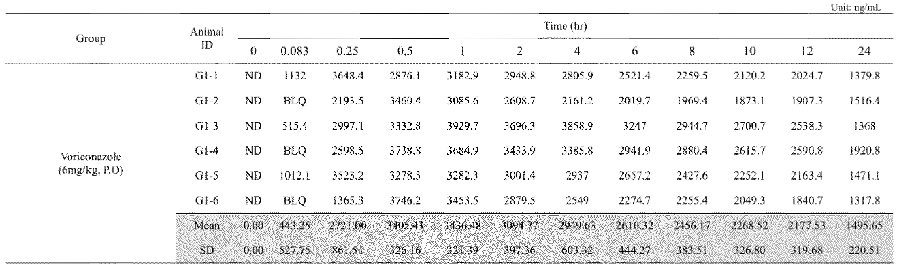

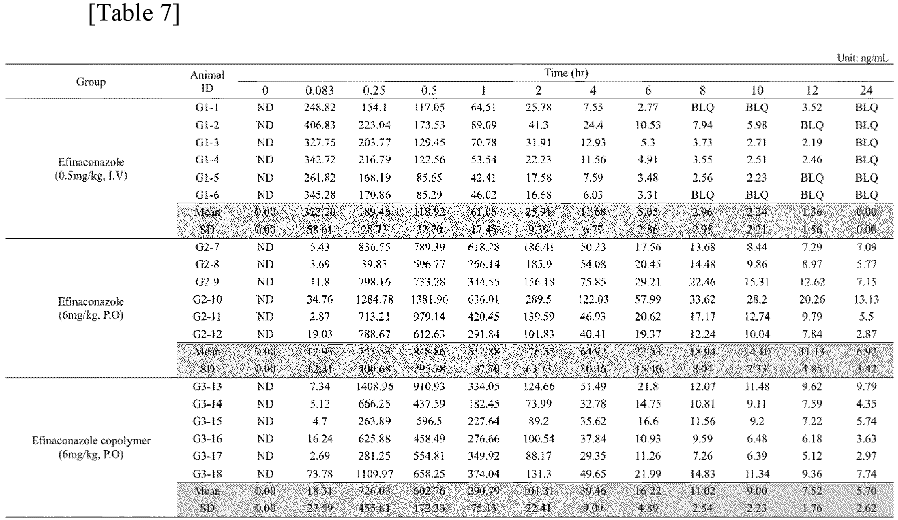

AUCt, a bioavailability parameter, is the area under the plasma concentration-time curve from the time of administration medication to the last time point of blood collection with a measurable concentration and was calculated by the trapezoidal formula. Cₘₐₓ is the highest plasma drug concentration read from the plasma drug concentration-time curve of each sample and Tₘₐₓ is the time taken to reach the highest plasma drug concentration read from the plasma drug concentration-time curve of each sample. AUC_{∞} is the area under the plasma concentration-time curve from the time of administration to time infinity. The calculated PK parameters of voriconazole and efinaconazole are shown in Table 8.

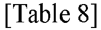

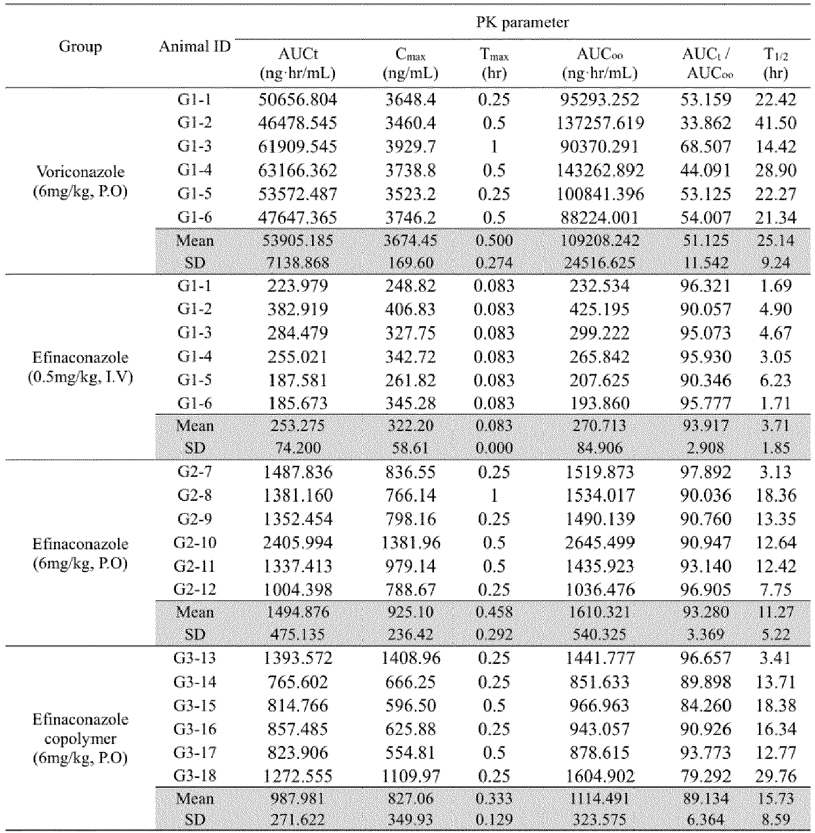

As can be seen from the results in Table 8, the voriconazole group (6 mg/kg, P.O), efinaconazole group (0.6 mg/kg, I.V), efinaconazole group (6 mg/kg, P.O), and efinaconazole copolymer group (6 mg/kg, P.O) were found to have the following PK parameters: AUCt values of 53,905.19±7,138.87, 253.28±74.20, 1,494.88±475.14, and 987.98 ± 271.62, respectively; AUC_{∞} values of 109,208.24±24,516.63, 270.71±84.91, 1,610.32±540.33, and 1,114.49±323.58, respectively; and Cₘₐₓ values of 3,674.45±169.60, 322.20±58.61, 925.10±236.42, and 827.06±349.93, respectively.

Statistical analysis revealed that the parameters Cₘₐₓ and AUCt of the efinaconazole copolymer group (6 mg/kg, P.O) were significantly low compared to those of the efinaconazole group (6 mg/kg, P.O) (see Tables 9-12).

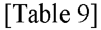

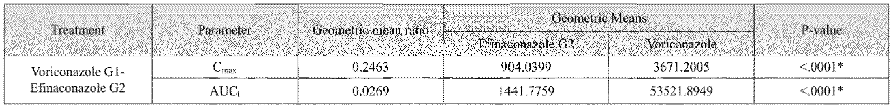

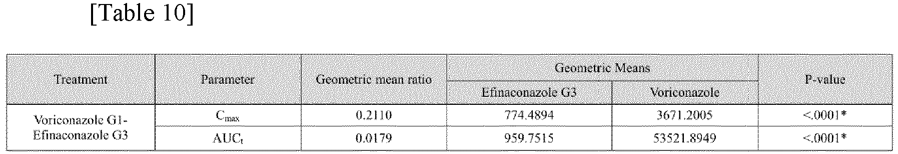

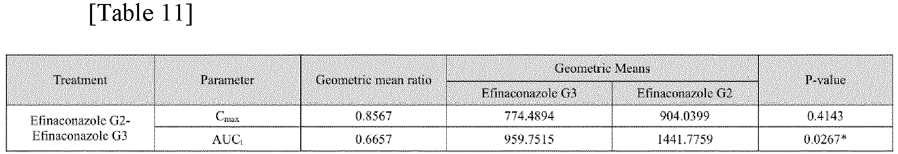

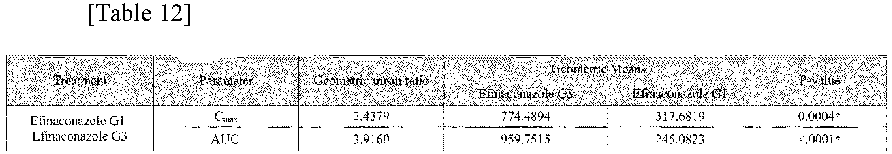

The bioavailabilities of the efinaconazole copolymer group (6 mg/kg, P.O) and the efinaconazole group (6 mg/kg, P.O) were found to be 35.69±22.61% and 51.36±24.63%, respectively (see Tables 13 and 14).

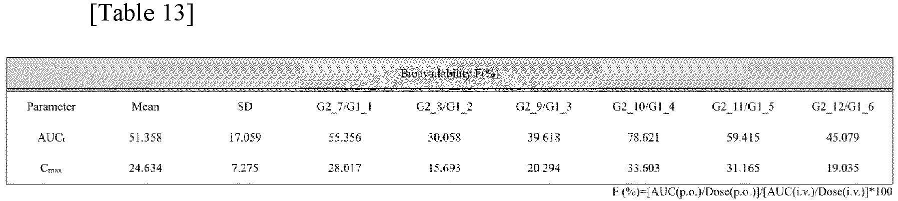

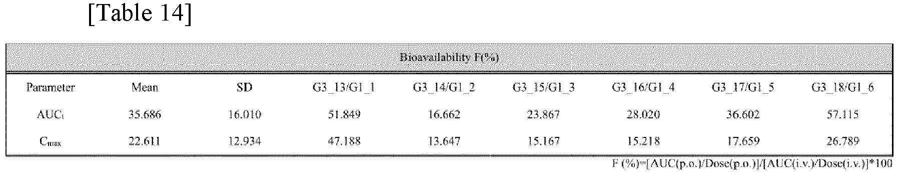

The AUCt level of the efinaconazole copolymer group (6 mg/kg, P.O) was found to be significantly low corresponding to 66% of that of the efinaconazole group (6 mg/kg, P.O) but is regarded as substantially exceeding that of the efinaconazole group considering that the content of the raw substance in the copolymer formulation was 50%

The bioavailability of the efinaconazole copolymer group (6 mg/kg, P.O) was 35.69%, which corresponds to ~70% of that of the efinaconazole group (6 mg/kg, P.O) (51.36%) under the premise that dose linearity is given, revealing that the degree of exposure of the efinaconazole copolymer (test substance) formulation in the body of the beagle dogs is much higher than the theoretically expected level (50%).

These test results concluded that the bioavailability of efinaconazole is higher than those of other antifungals, including voriconazole and itraconazole, when administered orally even at a low dose. Therefore, oral administration of efinaconazole is expected to be therapeutically very effective.

## Claims

1. A pharmaceutical composition for oral use comprising a co-formed product of efinaconazole and polyethylene glycol and a pharmaceutically acceptable additive.

2. The pharmaceutical composition according to claim 1, wherein the pharmaceutical composition is formulated into a tablet or solution.

3. The pharmaceutical composition according to claim 1, wherein the pharmaceutical composition comprises 0.6 to 2040 mg of the efinaconazole.

4. The pharmaceutical composition according to claim 1, wherein the weight ratio of the efinaconazole to the polyethylene glycol in the co-formed product is 1: 1.

5. The pharmaceutical composition according to claim 1, wherein the pharmaceutically acceptable additive is selected from the group consisting of excipients, binders, disintegrants, surfactants, lubricants, colorants, and mixtures thereof.

6. The pharmaceutical composition according to claim 5, wherein the excipients are lactose hydrate, microcrystalline cellulose, microcrystalline cellulose-lactose, methyl cellulose, ethyl cellulose, hydroxyethyl cellulose, hydroxypropyl cellulose, hydroxypropyl methyl cellulose, carboxymethyl cellulose salts, other substituted and unsubstituted celluloses, corn starch, pregelatinized starch, lactose, anhydrous lactose, sugar alcohols, sucrose, xylitol, acrylate polymers and copolymers, dextrates, dextrin, dextrose, maltodextrin, pectin, gelatin, inorganic diluents, and mixtures thereof.

7. The pharmaceutical composition according to claim 5, wherein the binders are polyvinyl alcohol-polyethylene glycol graft copolymers, polyvinylpyrrolidone vinyl acetate, ethyl cellulose, hydroxypropyl cellulose, hydroxypropyl methyl cellulose, carboxymethyl cellulose, methyl cellulose, polyvinyl alcohol, polyvinylpyrrolidone, polyacrylic acid, gelatin, propylene glycol, sodium alginate, and mixtures thereof.

8. The pharmaceutical composition according to claim 5, wherein the disintegrants are croscarmellose sodium, crospovidone, carboxymethyl cellulose salts, hydroxypropyl cellulose, microcrystalline cellulose, pregelatinized starch, sodium starch, glycolate, sodium starch glycolate, and mixtures thereof.

9. The pharmaceutical composition according to claim 5, wherein the lubricants are magnesium stearate, sodium stearyl fumarate, glyceryl behenate, and mixtures thereof.

10. The pharmaceutical composition according to any one of claims 1 to 9, wherein the pharmaceutical composition is used to prevent or treat a fungal infection.

11. The pharmaceutical composition according to claim 10, wherein the fungal infection is onychomycosis.
